# EUROPEAN PATENT SPECIFICATION

(11) **EP 1 687 062 B1**
(45) Date of publication and mention of the grant of the patent: **21.12.2011**
(21) Application number: 04794583.7
(22) Date of filing: 07.10.2004
(51) Int. Cl.: A61N 1/362

(54) **METHOD AND APPARATUS FOR CONTROLLING EXTRA-SYSTOLIC STIMULATION (ESS) THERAPY USING ISCHEMIA DETECTION**
VERFAHREN UND GERÄT ZUR KONTROLLE DER EXTRASYSTOLISCHEN STIMULATIONSTHERAPIE (ESS) MIT ISCHÄMIE-NACHWEIS
METHODE ET APPAREIL DESTINES A REGULER UN TRAITEMENT PAR STIMULATION EXTRASYSTOLIQUE (ESS) AU MOYEN D'UNE DETECTION D'ISCHEMIE

(30) Priority: 07.10.2003 US 680462
(43) Date of publication of application: 09.08.2006
(73) Proprietor: Medtronic, Inc., Minneapolis, MN 55432-5604 (US)
(72) Inventor: WARKENTIN, Dwight, H., Arden Hills, Minnesota 55112 (US); STADLER, Robert, W., Shoreview, Minnesota 55126 (US); ZILLMER, Glenn, C., Hudson, Wisconsin 54016 (US); DENO, D. Curtis, Andover, Minnesota 55304 (US)
(74) Representative: Hughes, Andrea Michelle
(86) International application number: PCT/US2004/033274
(87) International publication number: WO 2005/035052

(56) References cited:
- EP-A- 0 711 577
- WO-A-03/020364
- US-A- 6 058 328
- US-B1- 6 233 486
- US-B1- 6 363 279

## Description

The present invention relates generally to the field of implantable cardiac stimulation devices and more specifically to a device for delivering extra-systolic stimulation (ESS) and an associated method for detecting myocardial ischemia and controlling the delivery of ESS based on myocardial ischemia detection.

Decades ago so-called paired or coupled cardiac pacing was discovered and at least partially developed as an alternative to single stimulus cardiac pacing. Among the findings was that such pacing seemed to invoke a property of cardiac myocytes that causes enhanced mechanical function of the heart during depolarization events subsequent to delivery of an extra-systolic stimulus. The ESS may be delivered after either an intrinsic or pacing-induced systole. The magnitude of the enhanced mechanical function is strongly dependent on the timing of the ESS relative to the preceding intrinsic or paced systole. When correctly timed, an ESS pulse causes an electrical depolarization of the heart but the contribution to the attendant mechanical contraction for the cardiac cycle during which the ESS is delivered is absent or at least substantially weakened. The contractility of the subsequent cardiac cycles, referred to as the post-extra-systolic beats, is increased as described in detail in commonly assigned U.S. Pat. No. 5,213,098 issued to Bennett et al.

The mechanism of PESP is thought to involve the calcium cycling within the myocytes. The extra systole initiates a limited calcium release from the sarcolasmic reticulum (SR). The limited amount of calcium that is released in response to the extra systole is not enough to cause a normal mechanical contraction of the heart. After the extra systole, the SR continues to take up calcium with the result that subsequent depolarization(s) cause a large release of calcium from the SR, resulting in vigorous myocyte contraction.

The inventors hereof appreciate that the degree of mechanical augmentation on post-extra-systolic beats depends strongly on the time interval between a primary systole and the subsequent delivery of ESS, referred to herein as the "extra-systolic interval" (ESI). If the ESI is too long, the PESP effects are not achieved because a normal mechanical contraction takes place in response to the extra-systolic stimulus. As the ESI is shortened, a maximal effect is reached when the ESI is slightly longer than the myocardial refractory period. The cardiac cycle during which an ESS is applied, a measurable electrical depolarization occurs but without the expected attendant mechanical contraction (or with a relatively weakened contraction). When the ESI becomes too short, the ESS falls within the absolute refractory period of the myocardium to which the ESS was delivered and no depolarization occurs.

As indicated in the referenced '098 patent, delivery of ESS pulses to achieve subsequent stroke volume augmentation may increase the risk of arrhythmia induction. If the extra-systolic pulse is delivered during the vulnerable period, the risk of inducing tachycardia or fibrillation in arrhythmia-prone appears to increase even further. The vulnerable period encompasses the repolarization phase of the action potential, also referred to herein as the "recovery phase," and a period of time immediately following it. During the vulnerable period, the cardiac cell membrane is transiently hyper-excitable. It is therefore desirable to include cardioversion/defibrillation functions in an implantable device intended for delivering ESS therapy. Delivery of ESS pulses, however, may interfere with arrhythmia detection algorithms to some degree since additional blanking of sense amplifiers is required during ESS pulse delivery. The inventors appreciate that transient or prolonged myocardial ischemia is relatively common among HF patients, either as a causative effect or as a result of impaired myocardial perfusion due to decreased cardiac output. The risk of arrhythmias occurring during an acute myocardial ischemia event is well known. Thus, the presence of myocardial ischemia may exacerbate the risk of arrhythmias during delivery of ESS therapy.

Myocardial ischemia may be detected by noting changes to the EGM or ECG signal. In particular, T-wave and S-T segment changes are known to occur during a myocardial ischemia condition or as a result of the presence of a myocardial infarction (MI). A method for determining variation of S-T segment parameters using multiple cardiac electrogram signal vectors for determining physiological conditions such as ischemia is generally disclosed in U.S. Pat. No. 6,128,526 issued to Stadler et al., and in U.S. Pat. No. 6,397,100 issued to Stadler et al.

The effects of ESS therapy may advantageously benefit a large number of patients suffering from cardiac mechanical insufficiency, such as patients in HF, including congestive heart failure (collectively herein, "HF"). A need remains therefore for a clinically safe method for delivering an ESS therapy that achieves the mechanical benefits of while avoiding the risk of arrhythmias, particularly during ischemic episodes. In other cases, it may be desirable to prevent an acute degradation in cardiac performance during an episode of ischemia. The present invention is directed toward combining ischemia monitoring with ESS capabilities in an implantable cardiac stimulation device wherein ESS delivery is controlled based on ischemia monitoring results. One objective of the present invention is to address the need for reducing the risk of arrhythmias and/or the potential for under-detection of arrhythmias during an ischemic episode whether or not attributable to or occurring during delivery of an ESS therapy. In one embodiment of the present invention, delivery of ESS therapy is disabled or ESS control parameters are modified in response to an initial affirmative myocardial ischemia detection. In another embodiment of the present invention, ESS therapy begins in response to detection of a myocardial ischemia condition as an attempt to adequately re-perfuse the myocardium. In this way, ESS therapy does not contribute to an increased risk of arrhythmias and, moreover, does not interfere with the reliable performance of arrhythmia detection functions during an ischemic episode.

Another objective of the present invention is to provide mechanical enhancement of cardiac function during an episode of myocardial ischemia to reduce the likelihood of acute degradation of cardiac performance. Accordingly, in an alternate embodiment of the present invention, detection of an ischemic episode is responded to by initiating ESS therapy delivery or altering ESS control parameters so as to enhance cardiac mechanical function and thereby alleviate the ischemia or at least lessen the symptoms of ischemia.

The objectives of the present invention are realized in an implantable cardiac stimulation device capable of delivering ESS therapy and detecting myocardial ischemia and responding thereto. Additionally, the device is preferably capable of detecting and treating cardiac arrhythmias. In one embodiment, myocardial ischemia is detected by analysis of sensed cardiac electrical signals, *e.g*., changes in the ST segment or T-wave portion of a sensed cardiac electrogram (EGM). Myocardial ischemia may alternatively be detected based on latent evoked responses sensed following a stimulation pulse, which may be a primary pacing pulse or an extra-systolic pacing pulse. A relatively increased latency of evoked responses to ESS delivery may reflect slowed conduction due to myocardial ischemia or the presence of a myocardial infarction in the chamber receiving the ESS therapy.

In alternative embodiments, ischemia may be detected by monitoring metabolic indicators of ischemia, such as pH, oxygen saturation (including decreases in surrogates for oxygen saturation such as lactate, nitrogen peroxide, and the like) or other biochemical markers of myocardial ischemia. In yet other embodiments, ischemia is detected by monitoring a mechanical signal of cardiac function such as pressure or wall motion. Ischemia is detected based on slowed relaxation and/or contraction.

Upon detection of myocardial ischemia, the implantable device automatically adjusts ESS delivery by disabling ESS, modifying ESS control parameters, or initiating ESS. When ischemia is no longer detected, the device may automatically restore ESS according to normal operation conditions.
Figure 1A is an illustration of an implantable cardiac stimulation device, coupled to a set of leads implanted in a patient's heart, in which the present invention may be implemented.
Figure 1B is an illustration of an implantable cardiac stimulation device coupled to a set of leads implanted in a patient's heart which include a physiological sensor(s) for use in monitoring ischemia.
Figure 2 is a functional schematic diagram of the implantable medical device shown in Figure 1B.
Figure 3 is a flow chart providing an overview of a method for combining ischemia detection with ESS for improved safety.
Figure 4 is a flow chart providing an overview of a method for combining ischemia detection with ESS for enhancing cardiac performance during or after an ischemic episode.
Figure 5 is a flow chart summarizing steps performed in one embodiment of the present invention for detecting myocardial ischemia.
Figure 6 is a flow chart summarizing steps included in a method for combining ischemia monitoring based on conduction time measurements with ESS therapy.

The present invention is directed toward providing an implantable system for delivering an electrical stimulation therapy to achieve post extra-systolic cardiac augmentation, referred to herein as "extra systolic stimulation" (ESS) therapy, and for detecting myocardial ischemia. ESS therapy delivery is controlled based on the detection of myocardial ischemia, wherein ESS delivery may be suspended, initiated, or otherwise modified when ischemia is detected.

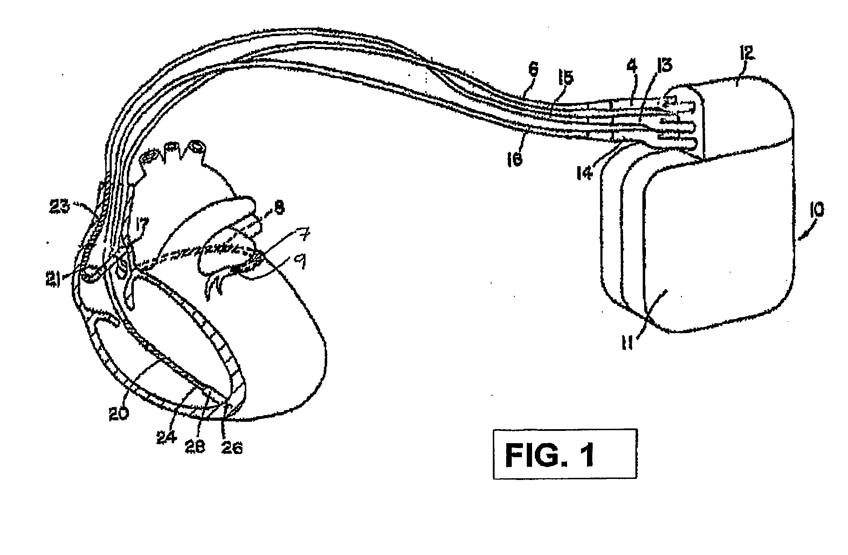

Figure 1A is an illustration of an exemplary cardiac stimulation device, referred to herein as an "implantable medical device" or "IMD," in which the present invention may be implemented. IMD 10 is coupled to a patient's heart by three cardiac leads. IMD 10 is capable of receiving cardiac signals and delivering electrical pulses for cardiac pacing, cardioversion and defibrillation. IMD 10 includes a connector block 12 for receiving the proximal end of a right ventricular lead 16, a right atrial lead 15 and a coronary sinus lead 6, used for positioning electrodes for sensing and stimulating in three or four heart chambers.

In Figure 1A, the right ventricular lead 16 is positioned such that its distal end is in the right ventricle for sensing right ventricular cardiac signals and delivering electrical stimulation therapies in the right ventricle which includes at least ESS and may include bradycardia pacing, cardiac resynchronization therapy, cardioversion and/or defibrillation. For these purposes, right ventricular lead 16 is equipped with a ring electrode 24, a tip electrode 26, optionally mounted retractably within an electrode head 28, and a coil electrode 20, each of which are connected to an insulated conductor within the body of lead 16. The proximal end of the insulated conductors are coupled to corresponding connectors carried by connector 14 at the proximal end of lead 16 for providing electrical connection to IMD 10.

The right atrial lead 15 is positioned such that its distal end is in the vicinity of the right atrium and the superior vena cava. Lead 15 is equipped with a ring electrode 21, a tip electrode 17, optionally mounted retractably within electrode head 19, and a coil electrode 23 for providing sensing and electrical stimulation therapies in the right atrium, which may include ESS and other cardiac stimulation therapies, such as bradycardia pacing, cardiac resynchronization therapy, anti-tachycardia pacing, high-voltage cardioversion and/or defibrillation. In one application of PESP, ESS is delivered in the atrial chambers to improve the atrial contribution to ventricular filling. The extra-systolic depolarization resulting from the atrial ESS stimulation pulse may be conducted to the ventricles for achieving PESP effects in both the atrial and ventricular chambers. The ring electrode 21, the tip electrode 17 and the coil electrode 23 are each connected to an insulated conductor with the body of the right atrial lead 15. Each insulated conductor is coupled at its proximal end to a connector carried by connector 13.

The coronary sinus lead 6 is advanced within the vasculature of the left side of the heart via the coronary sinus and great cardiac vein. The coronary sinus lead 6 is shown in the embodiment of Figure 1A as having a defibrillation coil electrode 8 that may be used in combination with either the coil electrode 20 or the coil electrode 23 for delivering electrical shocks for cardioversion and defibrillation therapies. Coronary sinus lead 6 is also equipped with a distal tip electrode 9 and ring electrode 7 for pacing and sensing functions and delivering ESS in the left ventricle of the heart. The coil electrode 8, tip electrode 9 and ring electrode 7 are each coupled to insulated conductors within the body of lead 6, which provides connection to the proximal bifurcated connector 4. In alternative embodiments, lead 6 may additionally include ring electrodes positioned for left atrial sensing and stimulation functions, which may include ESS and/or other cardiac stimulation therapies.

The electrodes 17 and 21, 24 and 26, and 7 and 9 may be used in sensing and stimulation as bipolar pairs, commonly referred to as a "tip-to-ring" configuration, or individually in a unipolar configuration with the device housing 11 serving as the indifferent electrode, commonly referred to as the "can" or "case" electrode. Preferably, IMD 10 is capable of delivering high-voltage cardioversion and defibrillation therapies. As such, device housing 11 may also serve as a subcutaneous defibrillation electrode in combination with one or more of the defibrillation coil electrodes 8, 20 or 23 for defibrillation of the atria or ventricles.

For the purposes of detecting myocardial ischemia, an EGM signal may be sensed from a bipolar "tip-to-ring" sensing vector, a unipolar tip-to-can sensing vector, a unipolar tip-to-coil or ring-to-coil sensing vector, or a relatively more global coil-to-can sensing vector. Any combination of available electrodes may be selected for sensing an EGM signal for detecting ischemia based on changes in the EGM signal.

It is recognized that alternate lead systems may be substituted for the three lead system illustrated in Figure 1A. For example, lead systems including one or more unipolar, bipolar and/or mulitpolar leads may be configured for sensing an EGM signal from which ischemia may be detected and for delivering ESS. Furthermore, epicardial leads could be substituted for transvenous leads. It is contemplated that extra-systolic stimuli may be delivered at one or more sites within the heart. Accordingly, lead systems may be adapted for sensing an EGM signal at multiple cardiac sites for detection of local ischemia and/or delivering extra-systolic stimuli at the multiple sites.

It is further recognized that subcutaneous ECG electrodes, incorporated on the device housing 11 or on subcutaneous leads extending therefrom, could be included in the implantable system and that myocardial ischemia may be detected from the subcutaneous ECG signals. An implantable system having electrodes for subcutaneous measurement of an ECG is generally disclosed in commonly assigned U.S. Pat. No. 5,987,352 issued to Klein. In alternative embodiments, multiple subcutaneous electrodes incorporated on the device housing 11 and/or positioned on subcutaneous leads extending from IMD 10 may be used to acquire multiple subcutaneous ECG sensing vectors for ischemia detection. Multi-electrode ECG sensing in an implantable monitor is described in U.S. Pat. No. 5,313,953 issued to Yomtov, et al.

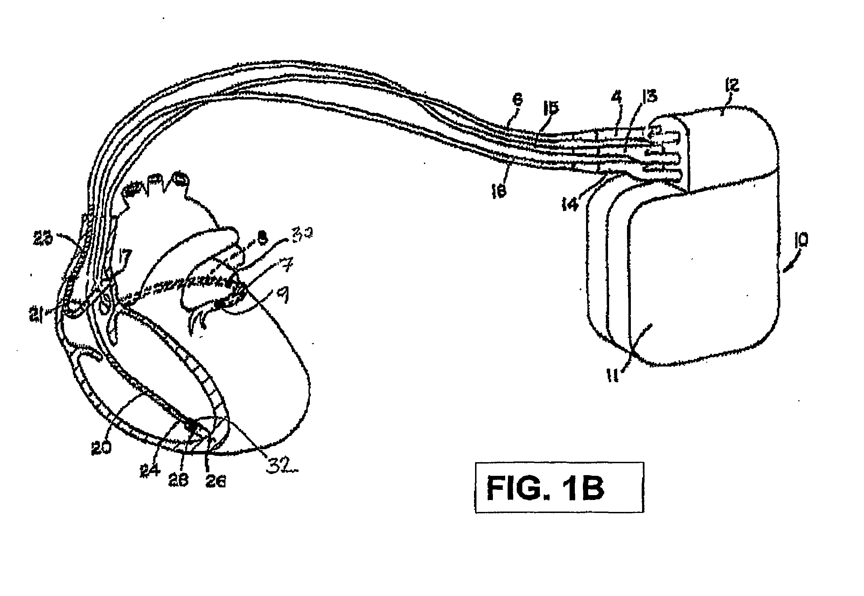

Figure 1B is an illustration of an IMD coupled to a set of leads implanted in a patient's heart that include alternative physiological sensor(s) for use in detecting myocardial ischemia. Biochemical sensor signals may be used alternatively to, or in addition to, electrical signals for myocardial ischemia detection. In Figure 1B, coronary sinus lead 6 is equipped with a biochemical sensor 30 capable of generating a signal relating to biochemical changes in the blood indicative of myocardial ischemia. Sensor 30 may be embodied as a pH sensor, oxygen saturation sensor, carbon dioxide sensor, or other biochemical sensor known in the art for sensing the level of biochemical markers that are indicative of myocardial ischemia. Colorimetric, fiber optic sensors for measuring pH, carbon dioxide, and/or other chemical parameters of the blood which may be usefully implemented for ischemia detection are generally disclosed in U.S. Pat. No. 5,047,208 issued to Schweitzer et al. A blood oxygen saturation sensor for use in myocardial ischemia detection may be embodied as a two wave length reflectance oximeter for determining oxygen saturation is generally disclosed in U.S. Pat. No. 4,813,421 issued to Baudino, et al.

Alternatively or additionally, a physiological sensor employed in detecting myocardial ischemia may be provided as a mechanical sensor. In the embodiment shown in Figure 1B, RV lead 16 is further equipped with a physiological sensor 32, which may be a mechanical sensor, for use in detecting myocardial ischemia. Early detectable sequelae of myocardial ischemia are depressed relaxation and contractile function of the ventricles. Therefore, by detecting a change in the mechanical function of the ventricles, myocardial ischemia may be tentatively diagnosed. Sensor 32 may be embodied, for example, as a pressure sensor for measuring the rate of pressure development (dP/dt) or an accelerometer for measuring ventricular wall motion. The rate of pressure development or acceleration and/or the rate of pressure decline or relaxation may be measured and compared to previous measurements or predetermined thresholds for detection of ischemia.

When sensor 32 is embodied as a pressure sensor, it may take the form of the lead-based pressure sensor generally disclosed in commonly-assigned U.S. Pat. No. 5,564,434 issued to Halperin et al. When sensor 32 is embodied as a wall motion sensor, it may take the form of a lead-based accelerometer for measuring wall motion as generally disclosed in U.S. Pat. No. 5,628,777 issued to Moberg, or U.S. Pat. No. 5,549,650 issued to Bornzin et al.

While ischemia detection methods may employ a single sensor signal, *i.e*. an electrical signal, a mechanical signal, or a biochemical signal, it is recognized that an ischemia detection algorithm may alternatively rely on two or more sensor signals, which may be a combination of one or more electrical, mechanical, and/or biochemical signals. For example, an intracardiac catheter, including electrical sensing means and pressure sensing means, and a method for detecting and diagnosing myocardial ischemia is generally disclosed in U.S. Pat. No. 5,025,786, issued to Siegel.

The use of two or more sensor signals for detecting myocardial ischemia may improve the specificity of ischemia detections.

The location of leads and corresponding sensors depicted in Figures 1A and 1B demonstrate approximate locations of a particular lead system, however, the positioning of leads and corresponding sensors may vary with respect to the heart anatomy according to the particular lead system used, types of sensors employed, and individual patient need. While a particular multi-chamber IMD and lead system is illustrated in Figures I A and 1B, methodologies included in the present invention may be adapted for use with other single chamber, dual chamber, or multichamber cardiac stimulation devices that are intended for delivering ESS and optionally include other electrical stimulation therapy delivery capabilities such as bradycardia pacing, cardiac resynchronization therapy, anti-tachycardia pacing, high-voltage cardioversion, and/or defibrillation.

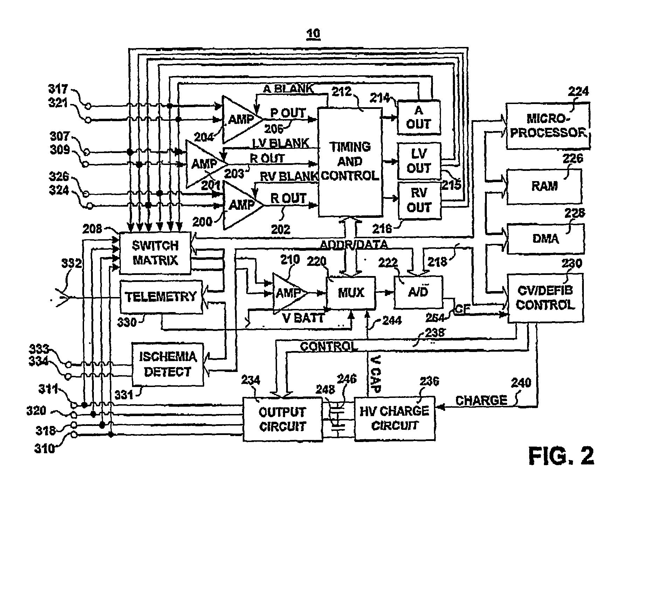

A functional schematic diagram of IMD 10 is shown in Figure 2. This diagram should be taken as exemplary of the type of device in which the invention may be embodied and not as limiting. The disclosed embodiment shown in Figure 2 is a microprocessor-controlled device wherein the functions of IMD 10 are controlled by firmware and programmed software algorithms stored in associated RAM and ROM carried out by a central processing unit of a typical microprocessor core architecture. Another microprocessor controlled implantable device in which the present invention may be implemented is disclosed in U.S. Pat. No. 6,438,408 issued to Mulligan et al. In addition, PCT application (publication no. WO 02/053026) by Deno et al., discloses an implantable medical device for delivering post extra-systolic potentiation stimulation. It is understood, however, that the methods of the present invention may also be practiced in other types of devices such as those employing custom integrated circuitry for performing specific device functions.

With regard to the electrode system illustrated in Figure 1B, IMD 10 is provided with a number of connection terminals for achieving electrical connection to the leads 6, 15, and 16 and their respective electrodes. The connection terminal 311 provides electrical connection to the housing 11 for use as the indifferent electrode during unipolar stimulation or sensing. The connection terminals 320, 310, and 318 provide electrical connection to coil electrodes 20, 8 and 23 respectively. Each of these connection terminal 311, 320, 310, and 318 are coupled to the high voltage output circuit 234 to facilitate the delivery of high energy shocking pulses to the heart using one or more of the coil electrodes 8, 20, and 23 and optionally the housing 11. Connection terminals 311, . 320, 310 and 318 are further connected to switch matrix 208 such that the housing 11 and respective coil electrodes 20, 8, and 23 may be selected in desired configurations for various sensing and stimulation functions of IMD 10.

The connection terminals 317 and 321 provide electrical connection to the tip electrode 17 and the ring electrode 21 positioned in the right atrium. The connection terminals 317 and 321 are further coupled to an atrial sense amplifier 204 for sensing atrial signals such as P-waves. The connection terminals 326 and 324 provide electrical connection to the tip electrode 26 and the ring electrode 24 positioned in the right ventricle. The connection terminals 307 and 309 provide electrical connection to tip electrode 9 and ring electrode 7 positioned in the coronary sinus. The connection terminals 326 and 324 are further coupled to a right ventricular (RV) sense amplifier 200, and connection terminals 307 and 309 are further coupled to a left ventricular (LV) sense amplifier 201 for sensing right and left ventricular signals, respectively.

The atrial sense amplifier 204 and the RV and LV sense amplifiers 200 and 201 preferably take the form of automatic gain controlled amplifiers with adjustable sensing thresholds. The general operation of RV and LV sense amplifiers 200 and 201 and atrial sense amplifier 204 may correspond to that disclosed in U.S. Pat. No. 5,117,524, by Keimel, et al. Generally, whenever a signal received by atrial sense amplifier 204 exceeds an atrial sensing threshold, a signal is generated on output signal line 206. P-waves are typically sensed based on a P-wave sensing threshold for use in detecting an atrial rate. Whenever a signal received by RV sense amplifier 200 or LV sense amplifier 201 that exceeds an RV or LV sensing threshold, respectively, a signal is generated on the corresponding output signal line 202 or 203. R-waves are typically sensed based on an R-wave sensing threshold for use in detecting a ventricular rate.

Switch matrix 208 is used to select which of the available electrodes are coupled to a wide band amplifier 210 for use in digital signal analysis. Selection of the electrodes is controlled by the microprocessor 224 via data/address bus 218. The selected electrode configuration may be varied as desired for the various sensing, pacing, cardioversion, defibrillation and ESS functions of the IMD 10. Signals from the electrodes selected for coupling to bandpass amplifier 210 are provided to multiplexer 220, and thereafter converted to multi-bit digital signals by A/D converter 222, for storage in random access memory 226 under control of direct memory access circuit 228. Microprocessor 224 may employ digital signal analysis techniques to characterize the digitized signals stored in random access memory 226 to recognize and classify the patient's heart rhythm employing any of the numerous signal processing methodologies known in the art.

In some embodiments of the present invention, any available electrodes may be selected via switch matrix 208 for use in detecting myocardial ischemia employing digital signal analysis methods applied to the EGM signal(s) received from the selected sensing vectors. Signal analysis methods used for detecting myocardial ischemia from S-T segment or T-wave changes may be analogous to well-established methods known for use in ECG monitoring. Methods for monitoring changes in the EGM signal for use in ischemia detection are generally disclosed in the above-cited `526 and '100 patents issued to Stadler, et al.

Ischemia detection circuitry 331 may receive input from multiplexed signals from switch matrix 208 for use in analysis of one or more EGM sensing vector signals for the detection of ischemia. Ischemia detection circuitry 331 may alternatively or additionally receive signals from physiological sensors 30 and 32 via connection terminals 333 and 334. Ischemia detection circuitry may include signal conditioning circuitry, such as amplifiers, filters, rectifiers, etc. for conditioning a received signal and may further include processing circuitry for determining a signal parameter, such as a signal peak, a peak derivative or slope, an average, or other parameter that may be used by microprocessor 224 in detecting the presence of ischemia.

An ischemia detection parameter(s) may be determined from one or more cardiac cycles or a predetermined interval or time. In a preferred embodiment, a cardiac EGM signal is used to define cardiac cycle boundaries, for example by measuring R-R intervals. Although it is possible to define cardiac cycle boundaries from mechanical signals, such as ventricular pressure, these signals may be less reliable during ESS because of the altered mechanical responses occurring during extra systoles and post-extra systoles and may be further altered due to ischemia.

Implementations of sensors and circuitry and/or algorithms for detecting ischemia may alternatively be embodied as generally disclosed in U.S. Pat. No. 5,531,768 issued to Alfemess, U.S. Pat. No. 5,199,428 issued to Obel, U.S. Pat. No. 6,233,486 issued to Ekwall et al., U.S. Pat. No. 6,021,350 issued to Mathson.

The telemetry circuit 330 receives downlink telemetry from and sends uplink telemetry to an external programmer, as is conventional in implantable anti-arrhythmia devices, by means of an antenna 332. Data to be uplinked to the programmer and control signals for the telemetry circuit are provided by microprocessor 224 via address/data bus 218. Received telemetry is provided to microprocessor 224 via multiplexer 220. Numerous types of telemetry systems known for use in implantable devices may be used. The remainder of the circuitry illustrated in Figure 2 is an exemplary embodiment of circuitry dedicated to providing ESS, bradycardia pacing, cardioversion and defibrillation therapies. The timing and control circuitry 212 includes programmable digital counters which control the basic time intervals associated with various single, dual or multi-chamber pacing modes or anti-tachycardia pacing therapies delivered in the atria or ventricles. Timing and control circuitry 212 also determines the amplitude of the cardiac stimulation pulses under the control of microprocessor 224.

During pacing, escape interval counters within timing and control circuitry 212 are reset upon sensing of RV R-waves, LV R-waves or atrial P-waves as indicated by signals on lines 202, 203,206, respectively. In accordance with the selected mode of pacing, pacing pulses are generated by atrial output circuit 214, right ventricular output circuit 216, and left ventricular circuit 215 upon expiration of an escape interval. The escape interval counters are reset upon generation of pacing pulses, and thereby control the basic timing of cardiac pacing functions, which may include bradycardia pacing, cardiac resynchronization therapy, and anti-tachycardia pacing.

Atrial and ventricular sense amplifiers 200,201,204 are isolated from output circuits 214, 215,216 by appropriate isolation switches within switch matrix 208 and also by blanking circuitry operated by A-BLANK and RV-BLANK, and LV BLANK signals at least during and optionally for a short time following delivery of a cardiac stimulation pulse. The blanking interval applied may depend on the sensing electrode configuration selected.

Timing and control circuitry 212 further controls the delivery of ESS pulses at selected extra-systolic intervals (ESIs) following either a sensed intrinsic systole or primary pacing pulse. The output circuits 214, 215 and 216 are coupled to the desired electrodes for delivering cardiac pacing or ESS pulses via switch matrix 208. The durations of the escape intervals, including ESIs, used for controlling the timing of stimulation pulse delivery are determined by microprocessor 224 via data/address bus 218.

The value of the count present in the escape interval counters when reset by sensed R-waves or P-waves can be used to measure R-R intervals and P-P intervals for detecting the occurrence of a variety of arrhythmias. The microprocessor 224 includes associated ROM in which stored programs controlling the operation of the microprocessor 224 reside. A portion of the memory 226 may be configured as a number of recirculating buffers capable of holding a series of measured intervals for analysis by the microprocessor 224 for predicting or diagnosing an arrhythmia. Methods for detecting and classifying cardiac arrhythmias are well-known in the art. Reference is made, for example, to U.S. Pat. No. 5,545,186 issued to Olson, et al.

In response to the detection of tachycardia, anti-tachycardia pacing therapy can be delivered by loading a regimen from microcontroller 224 into the timing and control circuitry 212 according to the type of tachycardia detected. In the event that higher voltage cardioversion or defibrillation pulses are required, microprocessor 224 activates the cardioversion and defibrillation control circuitry 230 to initiate charging of the high voltage capacitors 246 and 248 via charging circuit 236 under the control of high voltage charging control line 240. The voltage on the high voltage capacitors is monitored via a voltage capacitor (VCAP) line 244, which is passed through the multiplexer 220. When the voltage reaches a predetermined value set by microprocessor 224, a logic signal is generated on the capacitor full (CF) line 254, terminating charging. The defibrillation or cardioversion pulse is delivered to the heart under the control of the timing and control circuitry 212 by an output circuit 234 via a control bus 238. The output circuit 234 determines the electrodes used for delivering the cardioversion or defibrillation pulse and the pulse wave shape.

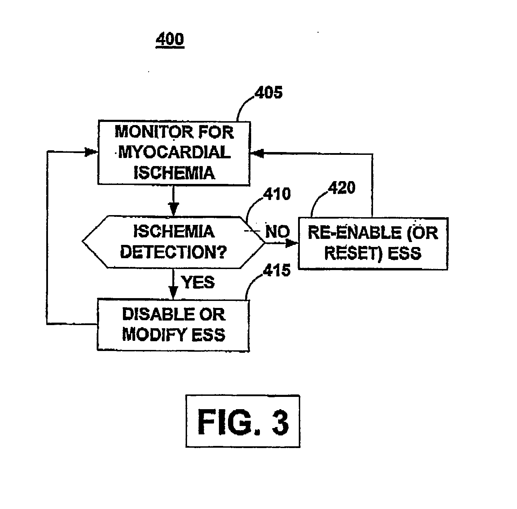

Figure 3 is a flow chart providing an overview of a method for combining ischemia detection with ESS for improved safety. In one embodiment of the present invention, ischemia monitoring is performed to detect when the risk of arrhythmias is increased and, as a result of such detection, allows ESS to be modified or suspended. Concern that ESS may inadvertently induce an arrhythmia if not carefully timed outside the vulnerable period, particularly when the myocardial substrate has an increased propensity for arrhythmias due to the presence of ischemia, may be alleviated by disabling ESS during ischemia. As such, method 400 includes a step 405 for monitoring for myocardial ischemia. As described above, ischemia may be detected based on an electrical, mechanical, or biochemical sensor signal according to methods known in the art. Ischemia monitoring may be performed on a continuous or sampled basis.

If a myocardial ischemia detection is made, according to decision step 410, ESS may be disabled or ESS control parameters may be modified at step 415. ESS control parameters may include, but are not limited to, the rate of ESS pulse delivery relative to the underlying intrinsic or paced cardiac rate, as will be described in greater detail below, or the ESI. ESS may be in progress at the time of the ischemia detection at step 410 and subsequently suspended at step 415, or ESS control parameters may be modified such that ESS continues but at adjusted pulse delivery control parameters. In other situations, ESS may not be in progress at the time of ischemia detection, but any ESS therapies scheduled or triggered to occur after the ischemia detection is made may be canceled or modified at step.

In some embodiments, ESS may be temporarily disabled until ischemia is no longer detected. After disabling or modifying ESS at step 415, myocardial ischemia monitoring may continue by returning to step 405. If ischemia is no longer detected at decision step 415, ESS may be re-enabled at step 420, or ESS control parameters may be reset to normal operating parameters. Method 400 then returns to step 405 to continue monitoring for myocardial ischemia, while ESS therapy is delivered according to normal operating parameters.

Alternatively, ESS may be permanently disabled or ESS control parameters may be permanently modified at step 415, until re-enabled or reset by a clinician, regardless if ischemia is no longer detected at step 410 during subsequent ischemia monitoring.

Disabling ESS during a detected episode of ischemia ensures that ESS does not contribute to the genesis of an arrhythmia during a period of increased arrhythmia risk due to myocardial ischemia. Furthermore, disabling ESS during ischemia ensures reliable function of arrhythmia detection algorithms by eliminating additional sense amplifier blanking intervals applied during ESS pulse delivery. Such blanking intervals may otherwise "blind" the implanted device from detecting high rates of intrinsic cardiac events that may meet arrhythmia detection criteria.

Alternatively, the ESS therapy may be modified rather than disabled during a detected ischemic episode to either reduce the likelihood of an arrhythmia induction or ensure proper performance of arrhythmia detection methods or both. ESS control parameters such as ESS rate or ESI may be adjusted. The ESS rate refers to the frequency of ESS pulses relative to the underlying heart rate. ESS pulses may be delivered following every sensed or primary paced systolic event such that extra systoles occur at a 1:1 ratio with the underlying paced or sensed heart rate. If ischemia is detected, ESS pulses may be delivered at a lower rate, such as with every other paced or sensed primary systolic event, every third event, every fourth event or some other ratio to the paced or sensed heart rate. By delivering ESS pulses less frequently than the primary paced or sensed rate, intrinsic cardiac events occurring at high rates, which might otherwise be "hidden" during sense amplifier blanking intervals applied during ESS pulse delivery, may still be reliably detected by the implanted device and used in classifying the heart rhythm.

Furthermore, the mechanical benefits of ESS can still be achieved at least to some degree, despite a lower ESS rate. The potentiation effect on post-extra-systolic beats will decay over several cardiac cycles, typically around six cardiac cycles. Therefore, an ESS pulse could be delivered following every second or third paced or sensed cardiac event, and the potentiation effect will normally persist long enough to still provide some mechanical enhancement on the intervening cardiac cycles, though to a somewhat lesser degree on later post-extra-systolic cardiac cycles than on the first post-extra-systolic cardiac cycle.

The ESI may be modified in addition to, or alternatively to, changing the ESS rate. When the primary concern regarding ESS delivery during myocardial ischemia is related to a potentially increased risk of inducing arrhythmias, the ESI may be lengthened to provide an added safety interval between the ESS pulse and the end of the vulnerable period. A consequence of increasing the ESI is likely to be a diminished mechanical enhancement on post-extra-systolic beats, however this tradeoff may be preferable to maintaining a short ESI when the underlying substrate is more arrhythmogenic.

By combining both an adjustment of the ESS rate and the ESI, both objectives of ensuring reliable arrhythmia detection and reducing the likelihood of an arrhythmia induction due to ESI may be achieved while still providing some hemodynamic benefit from ESS. Thus, the safety of administering ESS in a patient that may develop transient or prolonged myocardial ischemia is improved.

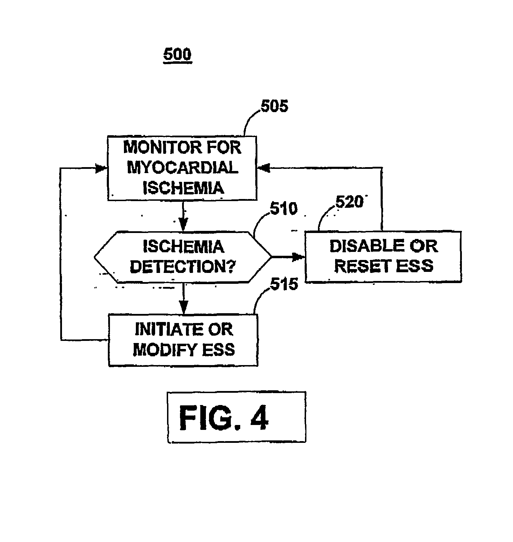

Figure 4 is a flow chart providing an overview of a method for combining ischemia detection with ESS for enhancing cardiac performance during or after an ischemic episode. In an alternative embodiment of the present invention, ischemia monitoring is performed to allow ESS to be initiated or modified in response to an ischemia detection in order to enhance cardiac performance and thereby potentially preclude acute decompensation which may lead to acute pulmonary edema, cardiac shock and even sudden death. By enhancing cardiac performance when ischemia is detected through the delivery of ESS, increased myocardial perfusion may alleviate or reverse the ischemia and restore stable function before severe consequences of myocardial ischemia can occur.

As such, method 500 includes step 505 for monitoring myocardial ischemia in the manner described above in conjunction with Figure 4. If a myocardial ischemia detection is made, according to decision step 510, ESS may be initiated if not already in progress and/or ESS control parameters may be modified at step 515.

In some embodiments, ESS may be initiated upon ischemia detection and delivered until ischemia is no longer detected or through a predefined interval of time thereafter. After initiating or modifying ESS at step 515, myocardial ischemia monitoring continues by returning to step 505. If ischemia is no longer detected at decision step 515, ESS may be terminated or the operating parameters may be reset at step 520 either immediately or after a defined interval of time. Method 500 then returns to step 505 to continue monitoring for myocardial ischemia, while ESS therapy may be delivered according to normal operating parameters.

Alternatively, ESS may be permanently enabled or ESS control parameters may be permanently modified at step 515, until disabled or reset by a clinician, regardless if ischemia is no longer detected at step 510 during subsequent ischemia monitoring. Initiating ESS during a detected episode of ischemia may act to alleviate or reverse the ischemic condition. If ESS is in progress when ischemia is detected, the ESS therapy may be modified in an attempt to provide even greater enhancement of mechanical cardiac function. For example, if the ESS rate is less than the underlying heart rate, the ESS therapy may be modified to be delivered at a higher rate during ischemia. During normal operation, ESS pulses may be delivered following every other sensed or paced primary systolic event during normal operation such that extra systoles occur at a 1:2 ratio with the underlying paced or sensed heart rate. It may be desirable to set the ESS rate to a rate less than the underlying cardiac rate during normal operation for a variety of reasons, *e.g.*, to conserve battery longevity. If ischemia is detected, however, ESS pulses may be delivered at a higher rate, such as with every paced or sensed primary systolic event so as to maximize the mechanical benefit of ESS in an attempt to increase myocardial perfusion. One method of increasing myocardial perfusion using ESS therapy delivery involves greatly increasing the diastolic fraction of time in the cardiac cycle. Because the coronaries are perfused during diastole, rhythms with a greater ratio of diastole to systole may lead to greater coronary perfusion. ESS therapy increases the diastolic/systolic ratio. ESS may additionally or alternatively be modified at step 515 by adjusting the ESI. As noted earlier, the degree of mechanical enhancement of post-extra-systolic beats depends on the length of the ESI. As the ESI is lengthened, the mechanical enhancement is decreased. It may not always be desirable to achieve maximum mechanical enhancement using a short ESI for long periods of time. Therefore, during normal ESS operation, an ESI may be set so as to provide some degree of beneficial mechanical enhancement that may be less than the maximum mechanical enhancement achievable using a shorter ESI. If ischemia is detected, however, a maximum mechanical enhancement may be desired in order to achieve the greatest improvement in myocardial perfusion. Therefore, a modification to ESS therapy that may be made at step 515 after detecting ischemia may be an adjustment of the ESI. By both increasing ESS rate (when the rate is less than the underlying heart rate) and shortening ESI up to a safe minimum limit for avoiding stimulation during the vulnerable period, an additive effect of enhanced mechanical function may be gained to thereby increase myocardial perfusion and alleviate ischemia or the symptoms of ischemia.

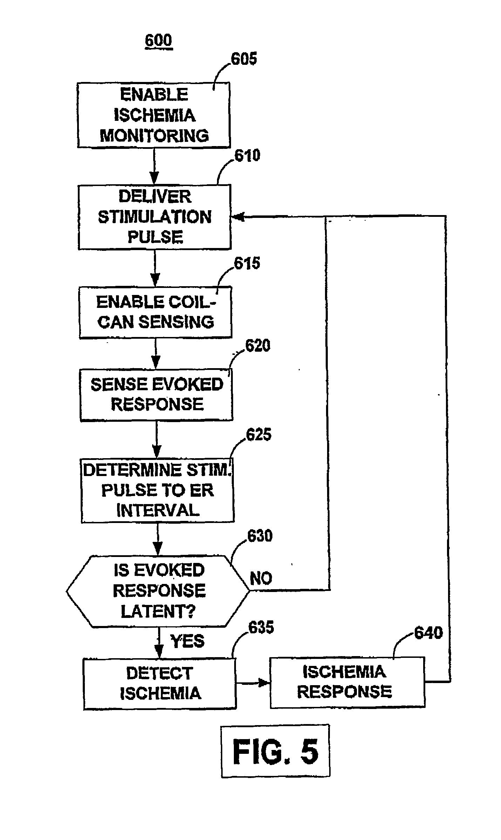

Figure 5 is a flow chart summarizing steps performed in one embodiment of the present invention for detecting myocardial ischemia. One known consequence of myocardial ischemia is a slowing of the electrical conduction rate through the ventricles. This decrease in conduction velocity may be measurable by sensing an evoked response at a distant endocardial or epicardial location following a cardiac stimulation pulse and noting changes in the interval between a stimulation pulse and the sensed evoked response. In this regard, non-provisional U.S. patent application serial no. 10/284,900 filed 31 October 2002 and entitled, "Ischemia Detection Based on Cardiac Conduction Time," is hereby incorporated by reference herein.

Myocardial ischemia monitoring is enabled at initiation step 605. Myocardial ischemia monitoring may be enabled for purposes of controlling ESS therapy delivery in accordance with the present invention or for other monitoring or therapy control purposes. At step 610, a cardiac stimulation pulse is delivered. The delivered pulse may be a primary pacing pulse delivered at a rate slightly greater than the intrinsic heart rate. Alternatively, the stimulation pulse delivered at step 610 may be an ESS pulse, delivered after the vulnerable period following an intrinsic or pacing-induced primary systole. In either case, the stimulation pulse is of sufficient energy to capture the heart. The stimulation pulse may be delivered using any available unipolar or bipolar pacing electrode pair. Preferably, the stimulation pulse is delivered in the right or left ventricle using an available pacing tip electrode paired with a ring electrode for bipolar stimulation or the device housing for unipolar stimulation.

At step 615, evoked response sensing is enabled preferably by selecting a coil-to-can sensing configuration. With respect to the lead system shown in Figures 1A and 1B, a preferred sensing configuration is RV coil 20 to housing 11 or LV coil 8 to housing 11, although other sensing vectors may be advantageously utilized (e.g., tip-to-ring, etc.). In addition, if the morphology of the resulting EGM waveforms rather than just the timing of fiducial points of the PQRST complex a vector from one of the coils 20,8 to housing 11 will likely produce superior results. If timing is of paramount importance, a tip-to-ring sensing vector should produce adequate results. At step 620, the evoked response following the stimulation pulse delivered at step 610 is sensed. Methods for evoked response sensing are well known in the art. Typically, the evoked response is sensed based on a threshold crossing of the selected EGM signal that occurs within a sensing window. The evoked response sensing window is generally set following the stimulation pulse corresponding to the time during which an evoked response is expected to occur. At step 625 the interval between the delivered stimulation pulse and sensed evoked response is measured as the conduction time.

At step 630 a comparative analysis of the measured conduction time is performed to determine if the evoked response is latent, which latency may be due to myocardial ischemia. The interval measured at step 625 may be compared to a predetermined threshold interval corresponding to a maximum expected conduction time under non-ischemic conditions. If the measured conduction interval exceeds this threshold, the evoked response may be considered latent at decision step 630.

Alternatively, the interval measured at step 625 may be compared to a running average or some other previously determined average conduction time. If the interval measured at step 625 is greater than a previously-determined average conduction time by a predetermined amount, the evoked response may be considered latent. In other embodiments, a conduction time trend may be determined at decision step 630 based on a number of consecutive conduction time interval measurements at step 625. If an increasing trend in conduction time is recognized, the evoked response is considered latent at decision step 630.

If the sensed evoked response is determined to be latent at step 630, ischemia is detected at step 635. An ischemia response may optionally be provided at step 645. An ischemia response may be storage of the ischemia detection time and date along with other desired physiological or device-related data, or the initiation or modification of a therapy. In accordance with the present invention, an ischemia response may be a withholding, modification or initiation of ESS therapy.

If the sensed evoked response is not latent, method 600 may return to step 620 to continue monitoring for myocardial ischemia by measuring the conduction time between a stimulation pulse and a sensed evoked response. Ischemia monitoring may continue until disabled or until ischemia is detected and an ischemia response is executed. Ischemia monitoring may continue after an ischemia response by returning to step 610 in order to detect a reversal of ischemia based on a restoration of normal conduction time as evidenced by detecting an evoked response that is not determined to be latent.

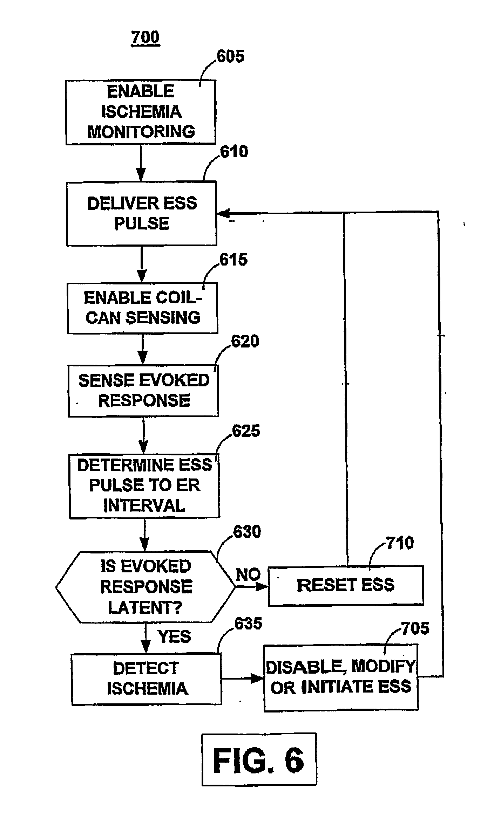

Figure 6 is a flow chart summarizing steps included in a method for combining ischemia monitoring based on conduction time measurements with ESS therapy. Identically numbered steps shown in method 700 of Figure 6 correspond to the same steps shown in ischemia monitoring method 600 of Figure 5. However, in method 700, an ESS pulse is delivered at step 610 such that the evoked response sensed at step 620 is an extra systole, and the conduction time interval measured at step 625 is the conduction time of the extra systole evoked by the ESS pulse and sensed by the coil-to-can sensing configuration.

The response to an ischemia detection made at step 635 involves the disabling of ESS, modification of ESS control parameters, or initiation of ESS at step 705. As described previously, disabling ESS or modification of ESS control parameters may be performed upon ischemia detection in order to safeguard against arrhythmia induction and/or ensure reliable performance of arrhythmia detection functions. Alternatively, ESS may be initiated in response to an ischemia detection and/or ESS control parameters may be modified in order to improve cardiac performance and in turn increase myocardial perfusion to thereby alleviate or reverse the ischemia or symptoms of ischemia. Following this response, myocardial ischemia monitoring continues. When myocardial ischemia is no longer detected based on recognition of a normal evoked response conduction time at step 630, ESS may be reset at step 710 according to normal operation conditions. Thus, at step 710, ESS may be resumed if previously disabled at step 705, ESS control parameters may be reset to normal operating parameters if adjusted previously at step 705 in response to ischemia detection, and/or ESS may be disabled if previously initiated at step 705.

The present invention may be embodied as executable instructions stored on a computer readable medium and operable under processor control. All suitable types of computer readable media are expressly intended to be covered hereby, as set forth in the appended claims.

A method and apparatus have been described herein for advantageously combining ischemia monitoring with ESS capabilities in an implantable device to achieve various benefits. The particular embodiments described herein are intended to be exemplary, rather than limiting, as the invention may be modified and practiced in different but equivalent manners apparent to those skilled in the art having the benefit of the teachings herein. Accordingly, the protection sought herein is set forth in the claims below.

## Claims

1. An apparatus for providing an adaptable extra-systolic stimulation (ESS) therapy delivery sequence in response to an output signal from a myocardial ischemia monitor, comprising:
means (30) for monitoring a volume of myocardial tissue for a myocardial ischemic condition and providing an output signal related to a presence or an absence of a myocardial ischemia condition;
means (224) for, in the event that the output signal indicates the presence of the myocardial ischemic condition and an ESS therapy is presently being applied to the volume of myocardial tissue, at least temporarily ceasing further delivery of the ESS therapy; and means for, in the event that the output signal indicates the presence of the myocardial ischemic condition and the ESS therapy is not presently being applied to the volume of myocardial tissue, initiate delivery of the ESS therapy.

2. An apparatus according to claim 1, further comprising:
means for, in the event that the output signal indicates the absence of the myocardial ischemic condition and an ESS therapy is presently being applied to the volume of myocardial tissue, then either continuing delivery of the ESS therapy, or mode-switching to another cardiac stimulation therapy modality.

3. An apparatus according to claim 1 or 2, further comprising means for determining a variation in:
a cardiac conduction interval from pacing pulse delivery to resultant depolarization over at least two different cardiac cycles,
a present sensor signal output compared to a prior sensor signal output, wherein said sensor couples to a portion of myocardial tissue and comprises one of: a mechanical sensor, a biological sensor, a metabolic sensor;
a variation in S-T segment parameters relative to an isoelectric baseline parameter, or
a variation in a portion of a T-wave of a PQRST complex,
using a cardiac electrogram signal vector for determining the onset, presence or absence of an ischemia condition.

4. An apparatus according to claim 3, wherein the cardiac electrogram vector comprises at least a one of: a tip-to-ring electrode vector, a coil-to-can electrode vector, a coil-to-coil electrode vector, a tip-to-can electrode vector, a ring-to-can electrode vector, a ring-to-ring vector.

5. An apparatus according to claim 4, further comprising:
means (330) for wirelessly transmitting the output signal to a remote device; or
means for vibrating the implantable pulse generator in relation to a state change in the output signal.

6. An apparatus according to claim 5, further comprising:
means for displaying an indicia by or on the remote device, wherein said indicia comprises a visual indicia related to the output signal.

7. An apparatus according to claim 5, wherein said remote device is coupled to a clinician information network.

## Patentansprüche

1. Vorrichtung zum Schaffen einer modifizierbaren verabreichungsfolge einer extrasystolischen Stimulationstherapie (ESS-Therapie) in Reaktion auf ein Ausgangssignal von einem Monitor für myokardiale Ischämie, mit:
Mitteln (30), um ein Volumen von Myokardialgewebe auf eine Bedingung einer myokardialen Ischämie zu überwachen und um ein Ausgangssignal, das mit dem Vorhandensein oder dem Fehlen einer Bedingung einer myokardialen Ischämie in Beziehung steht, bereitzustellen;
Mitteln (224), um in dem Fall, in dem das Ausgangssignal das Vorhandensein der Bedingung der myokardialen Ischämie angibt und eine ESS-Therapie momentan auf das Volumen des Myokardialgewebes angewendet wird, die weitere Verabreichung der ESS-Therapie zumindest vorübergehend zu beenden; und Mitteln, um in dem Fall, in dem das Ausgangssignal das Vorhandensein der Bedingung der myokardialen Ischämie angibt und die ESS-Therapie momentan nicht auf das Volumen des Myokardialgewebes angewendet wird, die Verabreichung der ESS-Therapie zu beginnen.

2. Vorrichtung nach Anspruch 1, ferner mit:
Mitteln, um in dem Fall, in dem das Ausgangssignal das Fehlen der Bedingung der myokardialen Ischämie angibt und eine ESS-Therapie momentan auf das Volumen des Myokardialgewebes angewendet wird, entweder die Verabreichung der ESS-Therapie fortzusetzen oder eine Betriebsartumschaltung zu einer weiteren Herzstimulationstherapie-Modalität vorzunehmen.

3. Vorrichtung nach Anspruch 1 oder 2, ferner mit Mitteln zum Bestimmen einer Veränderung
eines Herzleitungsintervalls von einer Schrittmacher-Impulsverabreichung zu einer resultierenden Depolarisation über wenigstens zwei verschiedene Herzzyklen,
eines momentanen Sensorsignalausgangs im Vergleich zu einem früheren Sensorsignalausgang, wobei der Sensor mit einem Abschnitt von Myokardialgewebe gekoppelt ist und einen mechanischen Sensor oder einen biologischen Sensor oder einem metabolischen Sensor enthält;
bei einer Änderung in S-T-Segmentparametern in Bezug auf einen isoelektrischen Grundlinienparameter, oder
bei einer Änderung in einem Abschnitt einer T-Welle eines PQRST-Komplexes,
unter Verwendung eines Herzelektrogramm-Signalvektors zum Bestimmen des Einsetzens, Vorhandenseins oder Fehlens einer Ischämiebedingung.

4. Vorrichtung nach Anspruch 3, wobei der Herzelektrogramm-Vektor wenigstens einen der folgenden Vektoren enthält: einen Spitze-zu-Ring-Elektroden-Vektor, einen Spule-zu-Dose-Elektroden-Vektor, einen Spule-zu-Spule-Elektroden-Vektor, einen Spitze-zu-Dose-Elektroden-Vektor, einen Ring-zu-Dose-Elektroden-Vektor, einen Ring-zu-Ring-Vektor.

5. Vorrichtung nach Anspruch 4, ferner mit:
Mitteln (330) zum drahtlosen Senden des Ausgangssignals zu einer entfernten Vorrichtung; oder
Mitteln zum Versetzen des implantierbaren Impulsgenerators in Schwingungen in Bezug auf eine Zustandsänderung des Ausgangssignals.

6. Vorrichtung nach Anspruch 5, ferner mit:
Mitteln zum Anzeigen einer Markierung durch oder auf der entfernten Vorrichtung, wobei die Markierung eine visuelle Markierung enthält, die mit dem Ausgangssignal in Beziehung steht.

7. Vorrichtung nach Anspruch 5, wobei die entfernte Vorrichtung mit einem klinischen bzw. Keiniker-Datenverarbeitungsnetz gekoppelt ist.

## Revendications

1. Dispositif pour fournir une séquence d'administration d'une thérapie par stimulation extra systolique (ESS) adaptable en réponse à un signal de sortie en provenance d'un moniteur d'ischémie myocardique, comportant :
des moyens (30) pour surveiller un volume de tissu myocardique pour une condition d'ischémie myocardique et délivrer un signal de sortie relatif à la présence ou à l'absence d'une condition d'ischémie myocardique ;
des moyens (224) pour, dans l'éventualité où le signal de sortie indique la présence de la condition d'ischémie myocardique et une thérapie ESS est actuellement appliquée au volume de tissu myocardique, cesser au moins temporairement d'administrer davantage la thérapie ESS ; et des moyens pour, dans l'éventualité où le signal de sortie indique la présence de la condition d'ischémie myocardique et la thérapie ESS n'est actuellement pas appliquée au volume de tissu myocardique, initier l'administration de la thérapie ESS.

2. Dispositif selon la revendication 1, comportant en outre :
des moyens pour, dans l'éventualité où le signal de sortie indique l'absence de la condition d'ischémie myocardique et une thérapie ESS est actuellement appliquée au volume de tissu myocardique, poursuivre alors l'administration de la thérapie ESS, ou basculer de mode sur une autre modalité de thérapie par stimulation cardiaque.

3. Dispositif selon la revendication 1 ou 2, comportant en outre des moyens pour déterminer une variation dans :
un intervalle de conduction cardiaque depuis la délivrance d'une impulsion de stimulation jusqu'à la dépolarisation obtenue en résultat sur au moins deux cycles cardiaques différents,
une sortie de signal de capteur actuel comparée à une sortie de signal de capteur antérieur, ledit capteur étant couplé à une partie de tissu myocardique et comportant l'un parmi : un capteur mécanique, un capteur biologique, un capteur métabolique ;
une variation dans des paramètres de segment S-T par rapport à un paramètre de ligne de base isoélectrique, ou une variation dans une partie d'une onde T d'un complexe PQRST,
l'utilisation d'un vecteur de signal d'électrogramme cardiaque pour déterminer le début, la présence ou l'absence d'une condition d'ischémie.

4. Dispositif selon la revendication 3, dans lequel le vecteur d'électrogramme cardiaque comporte au moins l'un parmi : un vecteur d'électrode d'embout à anneau, un vecteur d'électrode de bobine à gaine, un vecteur d'électrode de bobine à bobine, un vecteur d'électrode d'embout à gaine, un vecteur d'électrode d'anneau à gaine, un vecteur d'anneau à anneau.

5. Dispositif selon la revendication 4, comportant en outre :
des moyens (330) pour transmettre d'une manière sans fil le signal de sortie à un dispositif distant ; ou
des moyens pour faire vibrer le générateur d'impulsions implantable en relation à un changement d'état du signal de sortie.

6. Dispositif selon la revendication 5, comportant en outre :
moyens pour afficher un indice par l'intermédiaire du dispositif distant ou sur celui-ci, ledit indice comportant un indice visuel associé au signal de sortie.

7. Dispositif selon la revendication 5, dans lequel ledit dispositif de commande est couplé à un réseau d'informations de cliniciens.
